# EUROPÄISCHE PATENTANMELDUNG

(11) **EP 1 087 034 A1**
(43) Veröffentlichungstag der Anmeldung: **28.03.2001**
(21) Anmeldenummer: 00119159.2
(22) Anmeldetag: 05.09.2000
(51) Int. Cl.: C23C 16/04, B05D 7/24, A61F 2/00

(54) **Plasmabeschichtungsverfahren und dreidimensionales Kunststoffsubstrat mit einer Beschichtung**

(30) Priorität: 22.09.1999 DE 19945299
(71) Anmelder: GfE Metalle und Materialien GmbH, 90012 Nürnberg (DE)
(72) Erfinder: Breme, Frank, 91341 Röttenbach (DE); Buttstädt, Johannes, 90556 Wachendorf (DE); Planck, Heinrich, Prof., 72622 Nürtingen (DE); Doser, Michael, Dr., 70794 Filderstadt (DE); Müller, Erhard, Dr., 70565 Stuttgart (DE)
(74) Vertreter: Patentanwälte Ruff, Beier und Partner

(57) **Zusammenfassung**

Plasmabeschichtungsverfahren, bei dem ein Substrat in eine Reaktionskammer eingebracht wird und anschließend in der Reaktionskammer eine Prozeßgas-Atmosphäre erzeugt wird. Es erfolgt dann eine Abfolge von mehreren Plasmaerzeugungen, wobei zwischen jeder Plasmaerzeugung jeweils erneut eine geeignete Prozeßgas-Atmosphäre erzeugt wird.

## Beschreibung

Die Erfindung betrifft ein Plasmabeschichtungsverfahren sowie ein dreidimensionales Kunststoffsubstrat mit einer unmittelbar an die Kunststoffoberfläche angrenzenden Metallbeschichtung. Dabei ist es aus der EP 0 897 997 A1 bekannt, ein Kunststoffsubstrat mittels Plasmabeschichtung mit einer dünnen, geschlossenen metallhaltigen Schicht zu beschichten. Eine solche Beschichtung wird auch PACVD (plasma activated chemical vapor deposition) genannt. Ein Beschichtungsverfahren ist z. B. in der EP 0881 197 A2 beschrieben, auf die Bezug genommen wird.

Bei dem aus der EP 0 897 997 A1 bekannten Plasmabeschichtungsverfahren wird das Substrat in eine Reaktionskammer eingebracht, in der Reaktionskammer eine Prozeßgasatmosphäre erzeugt und anschließend in dem verdampfte metallorganische Verbindungen enthaltenden Prozeßgas ein Plasma erzeugt. Dabei lagern sich u. a. im Prozeßgas-Plasma enthaltene Metallionen am Substrat an und bilden dort eine metallhaltige Schicht. Eine solche Schicht enthält Metallionen, die jedoch nicht in metallischer Phase vorliegen, sondern an andere schichtbildende Substanzen gebunden sind. Die Schicht kann z. B. eine Keramik sein.

Gemäß der EP 0 897 997 A1 wird so ein Kunststoffsubstrat mit einer metallhaltigen Schicht überzogen.

Ein Problem bei Plasmabeschichtungen bzw. bei plasmabeschichteten Substraten ist es, daß die Schichtdicke der durch den Kontakt mit dem Plasma aufgetragenen Schicht davon abhängig ist, ob die Stelle, die beschichtet werden soll eine hinreichende Exposition gegenüber dem Prozeßgas aufweist. Bei schlechter Expositionen gegenüber dem Prozeßgas, insbesondere bei Hinterschneidungen, Hohlräumen und geringen Innendurchmessern des Substrats, kann eine Unterbrechung der erzeugten Schicht auftreten.

### Aufgabe und Lösung

Aufgabe der Erfindung ist es, ein Plasmabeschichtungsverfahren zu entwickeln, das geeignet ist sehr gleichmäßige Schichdicken auf dem Substrat zu erzeugen, insbesondere auch dann, wenn das Substrat eine strukturierte Oberfläche und/oder komplizierte Formgebung besitzt. Diese Aufgabe wird dadurch gelöst, daß das gattungsgemäß zugrundegelegte Plasmabeschichtungsverfahren bzw. das gattungsgemäß zugrundegelegte metallbeschichtete Kunststoffsubstrat gemäß den kennzeichnenden Merkmalen der unabhängigen Ansprüche ausgebildet wird.

Ein erfindungsgemäßes Plasmabeschichtungsverfahren, bei dem ein Substrat in eine Reaktionskammer eingebracht wird und anschließend in der Reaktionskammer eine Prozeßgas-Atmosphäre erzeugt wird, ist gekennzeichnet durch eine Abfolge von mehreren Plasmaerzeugungen an demselben Substrat, wobei zwischen jeder Plasmaerzeugung jeweils erneut eine geeignete Prozeßgas-Atmosphäre erzeugt wird.

Gemäß einer Ausgestaltung der Erfindung erfolgt die Plasmaerzeugung jeweils für eine vorgegebene, vorzügsweise zwischen 1 Sekunde und 5 Sekunden liegenden Brenndauer.

Dabei ist es sowohl möglich Prozeßgas kontinuierlich der Reaktionskammer zuzuführen - also auch während der Plasma-Erzeugung - als auch eine diskontinuierliche Prozeßgas-Zufuhr vorzunehmen, wozu jeweils zwischen zwei Plasmaerzeugungen ein Befühlen der Reaktionskammer mit Prozeßgas erfolgt. Der hierzu erforderliche Gasaustausch zwischen nach der Plasma-Erzeugung verbleibendem Restgas und zuzuführendem Prozeßgas kann vorzugsweise durch Abpumpen des Restgases und anschließendes Wiederbefüllen der Reaktionskammer mit Prozeßgas erfolgen. Gemäß einer vorteilhaften Ausgestaltung der Erfindung kann beim Wiederbefüllen der Reaktionskammer mit Prozeßgas zunächst ein höherer als während der Plasmaerzeugung erwünschter Druck in der Reaktionskammer erzeugt werden, Durch Abpumpen von Prozeßgas wird dann der während der Plasma-Erzeugung erwünschte Druck in der Reaktionskammer erzeugt. Es ist aber auch möglich nach dem Abpumpen vom verbrauchtem Prozeßgas nur soviel frisches Prozeßgas zu zuführen, bis der gewünschte Gasdruck erreicht ist.

Die Anzahl der Plasmaerzeugungen, die vorgenommen werden, ist abhängig von der erwünschten Schichtdicke auf dem Substrat. Normalerweise reichen 20 bis 200 insbesondere 50 bis 100 Zyklen aus,

Gemäß vorteilhafter Ausgestaltung der Erfindung herrscht in der Reaktionskammer eine solche Temperatur, daß eine Beschichtung des Substrats nur während der Dauer des extern erzeugten Plasmas stattfindet. Dies ist der Fall, obgleich die zur Beschichtung führenden Reaktionen mit steigender Temperatur eine höhere Abscheiderate und eine stärker anhaftende Anlagerung der Schicht bildenden Substanzen. Ferner ist der Druck der Prozeßgas-Atmosphäre vorzugsweise kleine als 10 mbar. Er beträgt insbesondere ca. 1 mbar. Dadurch wird eine unerwünschte Erwärmung vermieden.

Ein erfindungsgemäßes dreidimensionales Kunststoffsubstrat mit einer unmittelbar an die Kunststoffoberfläche angrenzenden Metallbeschichtung ist gekennzeichnet durch eine Metallschicht gleichmäßiger Schichtdicke, wobei die Schichtdicke - anwendungsabhängig - von 5 nm bis 700 nm beträgt, insbesondere zwischen 10 und 100 Nannometern liegt. Die Schichtdickenabweichung, bezogen auf die gesamte beschichtete Oberfläche ist kleiner ± 30 % insbesondere kleiner ± 15 %, wobei über die gesamte zu beschichtende Oberfläche eine geschlossene Schicht gebildet wird.

Unter dreidimensional werden hier insbesondere Gegenstände verstanden, die einen nicht von zugeführtem Prozeßgas durchspülbaren Bereich aufweisen und/oder die in Strömungsrichtung so lang sind, daß sich die Konzentration an Ionen aus Precursorgas an der abströmenden Seite während des extern erzeugten Plasmas unter einen vorgegebenen Wert, insbesondere 50 %, der ursprünglichen Konzentration abgesenkt hat.

Vorzugsweise handelt es sich bei dem Kunststoffsubstrat um einen Körper mit unregelmäßiger Oberfläche. Er kann insbesondere Hinterschneidungen und/oder von Außen zugängliche Hohlräume aufweisen. Bei der Beschichtung kann es sich auch um eine wenigstens teilweise Innenbeschichtung handeln.

Gemäß Ausgestaltungen der Erfindung handelt es sich bei dem Kunststoffsubstrat um einen zum Kontakt mit dem menschlichen oder tierischen Körper bestimmten Gegenstand, beispielsweise um eine zur Implantation im Körper bestimmte Prothese, insbesondere um eine textile Prothese z. B., um eine Gefäßprothese.

In bevorzugter Ausbildung ist die Beschichtung so ausgebildet, daß sie diffusionsdicht ist. Sie wirkt insbesondere als Diffusionssperre für Stoffe aus dem Substrat oder als Diffusionsperre für Stoffe in das Substrat.

Gemäß bevorzugter Ausgestaltung der Erfindung beträgt die elektrische Leitfähigkeit der Metallschicht mindestens 10⁻⁴ 1/Ω cm. Die Metallschicht enthält dabei vorzugsweise wenigstens eines der Elemente Ti, Ta, Nb, Zr, Hf und N.

Weitere Ausgestaltungen können den Unteransprüchen entnommen werden. Im übrigen ist die Erfindung anhand der in der Zeichnung dargestellten Ausführungsbeispiele näher erläutert; dabei zeigt:
- Fig. 1: Das Flußdiagramm eines ersten erfindungsgemäßen Verfahrens;
- Fig. 2: das Flußdiagramm eines zweiten erfindungsgemäßen Verfahrens und
- Fig. 3: eine teilgeschnittene Darstellung eines erfindungsgemäßen, eine unmittelbar an die Kunststoffoberfläche angrenzende Metallbeschichtung aufweisendes dreidimensionales Kunststoffsubstrat.

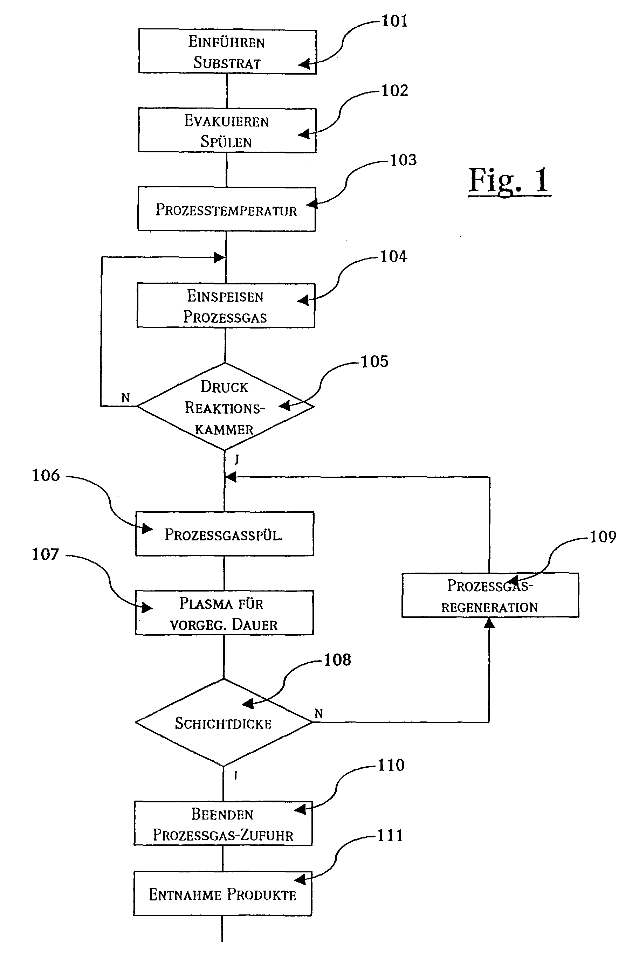

Die Fig. 1 zeigt das Flußdiagramm eines ersten erfindungsgemäßen Verfahrens, bei dem eine kontinuierliche Prozeßgaszufuhr stattfindet und bei dem zwischen zwei Plasmaerzeugungen eine Prozeßgas-Regeneration erfolgt.

Gemäß dem Schritt 101 wird das Substrat in eine Reaktionskammer eingeführt. Bei dem Substrat handelt es sich insbesondere um Kunststoffsubstrate, die zum Kontakt mit dem menschlichen oder tierischen Körper bestimmt sind. Der Kontakt kann entweder ein Blutkontakt, ein Kontakt mit Weichgewebe oder aber auch ein Knochenkontakt sein. Es kann sich bei dem Substrat beispielsweise um Polypropylen (PP)-Netze, beispielsweise zur Anwendung bei Hernien-Operationen oder um aus Kunststoffen hergestellte Prothesen, beispielsweise um Polyethylenterephthalat (PET) handeln.

Anschließend wird die Reaktionskammer gemäß dem Schritt 102 evakuiert. Dabei sollte ein Vakuum mit einem Druck, der höchstens, 10⁻² mbar beträgt, erzeugt werden. Anschließend wird die Reaktionskammer mit einem Inertgas gespült. Zum Spülen mit einem Inertgas - beispielsweise Stickstoff - wird in der Reaktionskammer durch Zufuhr dieses Inertgases eine Inertgasatmosphäre erzeugt, die einen höheren Druck aufweist. Anschließend wird durch Abpumpen des Inertgases wieder evakuiert. Während des Spülvorgangs kann, wenn gewünscht, schon ein Plasma erzeugt werden.

Gemäß dem Schritt 103 wird nun die Reaktionskammer auf Prozeßtemperatur gebracht. Hierzu kann mittels eines Heiztisches oder einer Halogenlampe die Reaktionskammer auf eine Temperatur von ca. 80° Celsius gebracht werden. Das Verfahren kann auch mit niederen Temperaturen durchgeführt werden (Zimmertemperatur bis 50° Celsius). Insbesondere dann, wenn eine besonders gute Anbindung der Beschichtung an das Substrat gewünscht ist, wird in höheren Temperaturbereich gearbeitet. Nunmehr wird gemäß dem Schritt 104 damit begonnen, Prozeßgas in die Reaktionskammer einzuspeisen. Das Einspeisen von Reaktionsgas kann beispielsweise mit einem Gasstrom von 3 l pro Minute erfolgen. Eine Reaktionskammer kann dabei eine Größe von ungefähr 50 l aufweisen. Als Trägergas kann insbesondere H₂ verwendet werden. Dem H₂ ist eine Precursorverbindung, eine metallorganische Verbindung in einer Konzentration (Beladung) von weniger als 0,05 mol/mol H₂ beigefügt. Im Schritt 105 wird dann überprüft, ob der Druck der Prozeßgasatmosphäre in der Reaktionskammer schon den gewünschten Druck zwischen 0,1 mbar und 10 mbar, vorzugsweise 1 mbar, erreicht hat. Ist dies nicht der Fall so wird weiterhin Prozeßgas zugeführt. Anderenfalls wird zum Schritt 106 übergegangen. Gemäß dem Schritt 106 setzt nun die kontinuierliche, mit festem Volumenstrom stattfindende Prozeßgaszufuhr in die Reaktionskammer statt, wobei der Volumenstrom des Prozeßgases nunmehr in einer Größenordnung von 0,1-10 l per Minute liegt. Gemäß dem Schritt 107 wird für eine vorgegebene Dauer ein Plasma erzeugt. Die Prozeßtemperatur von ca. 80° Celsius, die in dem Schritt 103 in der Reaktionskammer erzeugt wurde, ist so gewählt, daß die Schichtbildung nur während der Existenz des Plasmas stattfindet. Das Plasma kann entweder durch Einkopplung von elektromagnetischen Wellen, insbesondere im Bereich von 13,56 MHz in die Reaktionskammer oder aber durch Erzeugung eines Gleich- (DC) oder eines Wechsel-(AC)-Stromes erzeugt werden. Verfahren zur Erzeugung eines Plasmas in einem Gas, die hier verwendet werden können und die nicht zu einer wesentlichen Erwärmung in der Reaktionskammer führen sind aus dem Stand der Technik, beispielsweise aus der EP 0 897 997 A1 bekannt.

Das Plasma wird für eine vorbestimmte Zeit zwischen 1 und 5 Sekunden, beispielsweise für 2 Sekunden, erzeugt. Aufgrund der für Plasmaverfahren niederen Temperatur in der Reaktionskammer liegt ein Plasma nur solange vor, wie es aktiv durch Einkoppeln von Energie erzeugt wird. Während der Erzeugung des Plasmas wird das Substrat beschichtet. Während des Vorliegens eines Plasmas reduziert sich die Konzentration (Beladung) der Atome der metallorganischen Verbindung in der Prozeßgasatmosphäre auf ca. 1/5 des Ausgangswertes. Die Reduktion der Beladung in der Prozeßgasatmosphäre wird an den für das Prozeßgas schwer erreichbaren Stellen, wie Hohlräumen, Löchern und Hinterschneidungen durch die kontinuierliche Zufuhr von frischem Prozeßgas und ein entsprechendes zeitgleich stattfindendes Abpumpen des verbrauchten Prozeßgases aus der Reaktionskammer bei kontinuierlicher Plasmaerzeugung nicht kompensiert, da die erreichbare Gaszufuhr von frischem Prozeßgas wesentlich niedriger liegt als die Abnahme der Beladung bedingt durch die Reaktion des Prozeßgases und die Abscheidung des Metalls auf dem Substrat. Deshalb erfolgt die Plasmaerzeugung gemäß der Erfindung mit Unterbrechungen für die Wiederherstellung der gewünschten gleichmäßigen Prozeßgaskonzentration.

Nach Ablauf der vorgegebenen Dauer der Plasmaerzeugung wird gemäß dem Schritt 108 überprüft ob eine ausreichende Schichtdicke erzeugt wurde. Wurde eine ausreichende Schichtdicke nicht erreicht so wird gemäß dem Schritt 109 eine Prozeßgasregeneration durchgeführt. Dies geschieht dadurch, daß weiterhin kontinuierlich Prozeßgas mit ursprünglicher Beladung an metallorganischer Precursor-Substanz in die Reaktionskammer eingeführt wird. Gleichzeitig kann auch verbrauchtes Prozeßgas aus der Reaktionskammer herausgepumpt werden und dabei vorübergehend ein geringerer Druck in der Prozeßgas-Atmosphäre erzeugt werden, als dies während der Plasma-Erzeugung der Fall ist. Diese Maßnahme erhöht die Spülung mit Prozeßgas in schlecht durchströmten Bereichen, wie z.B. Hohlräumen und Hinterschneidungen. Dies wird solange, beispielsweise während eines vorgegebenen Zeitintervalls, durchgeführt bis die Prozeßgasatmosphäre in ausreichender Weise regeneriert ist. Die Regeneration der Prozeßgasatmosphäre bemißt sich an dem Druck der Atmosphäre in der Reaktionskammer sowie an der Beladung der Atmosphäre mit Präcursor-Verbindungen. Anschließend wird zum Schritt 106 gesprungen.

Wurde im Schritt 108 festgestellt, beispielsweise dadurch daß eine ausreichende Anzahl an Zyklen durchgeführt wurde, um die erwünschte Schichtdicke zu erreichen, wobei beispielsweise aus empirischen Versuchen bekannt ist, welche Schichtdicke während der einmaligen Erzeugung eines Plasmas unter den gegebenen Verhältnissen für die vorbestimmte Dauer auf dem Substrat abgeschieden wird. Ist dieser Wert bekannt, so muß lediglich mit der Anzahl der Plasma-Erzeugungen, die stattgefunden hat, multipliziert werden um die aufgebrachte Schichtdicke zu ermitteln. Hat diese den gewünschten Wert erreicht, so wird zum Schritt 110 gesprungen. Demgemäß wird die Zufuhr von frischem Prozeßgas beendet. Gemäß dem Schritt 111 wird nun die Reaktionskammer belüftet und das Produkt, das beschichtete Substrat, aus der Prozeßkammer entnommen.

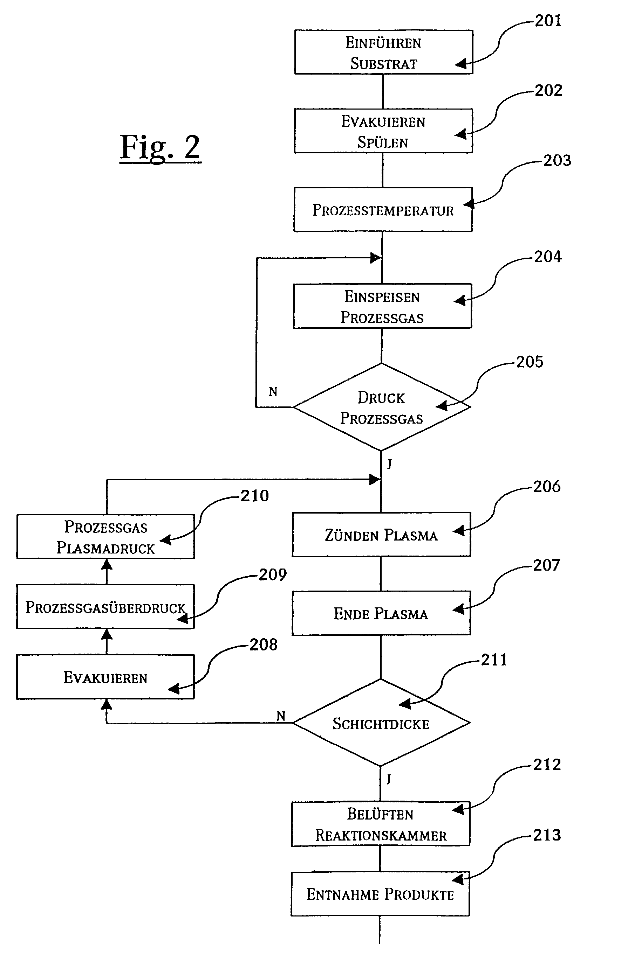

Die Fig. 2 zeigt das Flußdiagramm eines zweiten erfindungsgemäßen Verfahrens.

Dabei stimmen die Verfahrensschritte 201 bis 205 mit den in der Fig. 1 beschriebenen Verfahrenschritten 101 - 105 überein. Deshalb wird diesbezüglich auf die Beschreibung zu Fig. 1 verwiesen. Ebenso wie in den Verfahren gemäß der Fig. 1 kann das Spülen der Reaktionskammer gemäß dem Schritt 202 bzw. 102 mit einem Inertgas ersetzt werden durch eine Plasma-Reinigung von Substrat und Reaktionskammer sowie eine Aktivierung der Substratoberfläche. Hierzu wird in der Reaktionskammer eine Stickstoff-Atmosphäre erzeugt. In dieser Stickstoffatmosphäre wird, entsprechend der Vorgehensweise gemäß dem Schritt 107 bzw. den Schritten 206 und 207 für eine vorgegebene Zeitdauer ein Plasma erzeugt. Anschließend wird die Reaktionskammer evakuiert. Es wird dann zum Schritt 203 bzw. zum Schritt 103 übergegangen. Zum Evakuieren der Prozeßkammer kann in der Regel eine Saugpumpe verwendet werden.

Wurde im Schritt 205 festgestellt, daß der Druck des Prozeßgases den erwünschten Wert erreicht hat, so wird zum Schritt 206 übergegangen und das Plasma gezündet. Gemäß dem Schritt 207 wird die Plasmaerzeugung beendet, sobald die gewünschte Plasma-Erzeugungsdauer, die in der Regel zwischen 1 und 5 Sekunden beträgt, abgelaufen ist. In Schritt 211 wird die erreichte Schichtdicke an Hand empirischer Werte in Abhängigkeit der Beschichtungszyklen ermittelt.

Für die Einleitung des nachfolgenden Zyklus wird gemäß dem Schritt 208, beispielsweise mittels der Saugpumpe, die Reaktionskammer evakuiert. Es wird dann gemäß dem Schritt 209 in der Reaktionskammer eine Prozeßgasatmosphäre erzeugt, die einen höheren Druck aufweist, als die Atmosphäre, die erforderlich und gewünscht ist, um darin ein Plasma zu erzeugen. Es kann beispielsweise eine Prozeßgas-Atmosphäre erzeugt werden, deren Druck in der Reaktionskammer 20 mbar beträgt, wogegen während der Erzeugung eines Plasmas in der Reakionskammer lediglich eine Atmosphäre mit einem Druck von 1 mbar vorliegt. Die Überdruckerzeugung dient der Spülung der Prozeßkammer. Sie ist insbesondere dann von Vorteil, wenn das Substrat Hohlräume oder Hinterschneidungen aufweist oder wenn eine Innenbeschichtung eines Substrats durchzuführen ist, wobei die Durchströmung des Innenraumes mit Prozeßgas gering ist.

Anschließend wird gemäß dem Schritt 210 in der Reaktionskammer eine Prozeßgas-Atmosphäre erzeugt, deren Druck wieder auf den Druck reduziert wurde, der für die Plasma-Erzeugung erwünscht ist, beispielsweise 1 mbar. Sofern die Gestalt des Substrats nicht all zu sehr strömungsungünstig ist, kann es auch ausreichen, daß sich der Schritt 210 direkt an den Schritt 208 anschließt ohne daß die Erzeugung eines Überdrucks gemäß Schritt 209 stattfindet. Dann wird in dem Schritt 210 der Druck in der Reaktionskammer nach der Evakuierung soweit erhöht, daß der für die Erzeugung des Plasmas erwünschte Druck in der Prozeßgas-Atmosphäre vorliegt. Nachdem Schritt 210 wird zum Schritt 206 gesprungen.

Wird im Schritt 211 festgestellt, daß die Schichtdicke das gewünschte Maß hat so wird gemäß dem Schritt 212 die Reaktionskammer belüftet. Gemäß dem Schritt 213 kann das erzeugte Produkt, das nunmehr an seiner Oberfläche gleichmäßig mit Metall beschichtete Substrat, aus der Reaktionskammer entnommen werden.

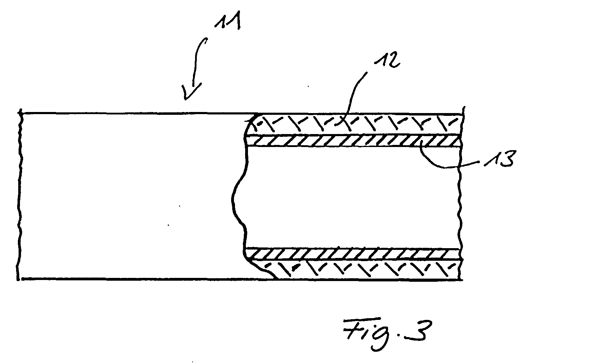

Die Fig. 3 zeigt in teilgeschnittener Darstellung ein Segment eines schlauchförmigen Substrates 12, daß auf seiner Innenseite mit einer metallhaltigen Schicht 13 überzogen wurde. Bei dem Substrat kann es sich beispielsweise um einen PVC-Schlauch handeln.

Bei PVC-Schläuchen, die als Schläuche für Herzlungen-Maschinen dienen, verhindert eine metallhaltige Beschichtung auf der Innenseite des Schlauches eine schädliche Diffusion von Weichmachern oder anderen Additiven aus dem Weich-PVC ins Blut. Zusätzlich wird die Blutverträglichkeit verbessert, wodurch es zu weniger schlauchinduzierten Trombosen kommt. Die Beschichtung hierfür kann bei einer kontinuierlichen Prozeßgaszufuhr und intervallartig eingeschaltetem Plasma durchgeführt werden. Vorzugsweise wird das Prozeßgas direkt in die Innenseite des Schlauches eingeleitet. Soll ein ca. 2 m langer Schlauch mit einem Innendurchmesser von 6 mm mit einer metallhaltigen Schicht im Schlauchinneren von ca. 30 nm beschichtet werden so kann dies dadurch erfolgen, daß der Schlauch in eine Reaktionskammer eingebracht wird. In der Reaktionskammer wird für die Beschichtung eine Atmosphäre eines Drucks von 1 mbar bei einer Temperatur von 80° Celsius erzeugt. Es findet eine kontinuierliche Zufuhr von Prozeßgas statt, wobei die Zufuhrgeschwindigkeit etwa 1 l_{N}/h beträgt wobei als Prozeßgas mit metallorganischen Verbindungen beladener Wasserstoff verwendet wird, das eine Beladung von 0,002 mol/mol H₂ der Precursor-Substanz Tetrakisdimethylaminotitan beinhaltet. Zur Beschichtung finden 100 Zyklen statt wobei während jedes Zyklus für 3 Sekunden ein Plasma erzeugt wird und danach während 2 Sekunden lediglich frisches Prozeßgas zugeführt wird.

Auch bei Gefäßprothesen aus PET (z.B. aus einem textilen Gewirke oder Gewebe aus PET-Fäden) kann eine metallhaltige Beschichtung durchgeführt werden, die sowohl Blutverträglichkeit als auch Biostabilität der Prothese verbessert. Es können besonders vorteilhaft nicht nur großlumige sondern auch kleinlumige Gefäßprothesen mit einem erfindungsgemäßen Verfahren beschichtet werden. Dabei kann die Beschichtung sowohl auf der Außenseite als auch auf der Innenseite der Gefäßprothese erfolgen, wobei die einzelnen Fasern der multifilen Fäden vollständig beschichtet werden. Durch Vibration kann das Gefüge der textilen Prothese gelockert werden, um die komplette Faseroberfläche auch an Überkreuzungsstelle zugänglich zu machen. Die Beschichtung verbessert dabei sowohl die Blutverträglichkeit als auch die Biostabilität der Prothese.

Um eine gleichmäßige Innen- und Außenbeschichtung zu erreichen ist es erforderlich eine gute Durchströmung der Reaktionskammer im Bereich der zu beschichtenden Substratoberfläche mit dem Prozeßgas zu erreichen, was vorzugsweise gemäß der Erfindung durch einen Druckwechsel in der Prozeßgas-Atmosphäre erreicht wird. Dabei kann sowohl eine Methode mit kontinuierlicher als auch die Methode mit diskontinuierlicher Zufuhr an Prozeßgas gewählt werden. Wird die Methode mit kontinuierlicher Zufuhr an Prozeßgas gewählt, so wird in der Phase der Prozeßgas-Regeneration, solange kein Plasma erzeugt wird, der Druck der Prozeßgas-Atmosphäre abgesenkt, während die Prozeßgaszufuhr konstant weiterläuft. Dies kann beispielsweise durch Erhöhung der Förderleistung der Saugpumpe - beispielsweise eine Rootspumpe - erfolgen. Hierzu muß lediglich die Pumpendrehzahl während der Regenerationsphase erhöht werden.

Eine Schichtdicke von ca. 50 nm kann in 50 Arbeitszyklen folgenden Ablaufs erreicht werden: Bei einer konstanten Prozeßgaszufuhr mit einem Volumenstrom V= 2 l_{N}/H wird H₂ mit einer Beladung von 0,002 mol_{TMT}/mol H₂ zugeführt. Die Prozeßtemperatur beträgt 120° Celsius, der Atmospärendruck während der Plasmaerzeugung 1 mbar. Dabei arbeitet die Saugpumpe während der Plasmaerzeugung - ein Plasma wird für 3 Sekunden erzeugt - lediglich bei 10 % ihrer maximalen Drehzahl. Nach dem Abschalten des Plasmas wird bei weiterhin kontinuierlich stattfindender Zufuhr von Prozeßgas der Druck der ProzeßgasAtmosphäre auf 10⁻² mbar abgesenkt, indem die Saugpumpe für ca. 10 Sekunden mit 100 % der Saugleistung betrieben wird. Anschließend wird die Saugleistung wieder auf 10 % der Nenndrehzahl reduziert. Nach einer Wartezeit von ca. 15 Sekunden stellt sich aufgrund der weiterhin konstant stattfindenden Prozeßgaszufuhr wieder einen Druck von 1 mbar ein. Nun kann erneut ein Plasma gezündet werden, womit der nächste Zyklus beginnt.

Weiterhin können auch Behältnisse aus Kunststoff beschichtet werden. Eine metallhaltige Beschichtung von Kunststoffen ist im Bereich der Aufbewahrung von Medikamenten, beispielsweise in Spritzen oder in Beuteln (Infusionsbeutel) vorteilhaft, wenn dadurch die Gasdurchlässigkeit des Kunststoffsubstrats verringert wird und daher oxidationsempfindliche Medikamente länger in dem Kunststoffbehältnis aufbewahrt werden können. Die Beschichtung erfolgt dabei vorzugsweise sowohl an der Innen- als auch an der Außenseite des Behältnisses. Damit sowohl im Innenraum des Behältnisses als auch auf der Außenseite des Behältnisses während der Plasmaerzeugung stets eine geeignete gleichmäßige Prozeßgasatmosphäre vorliegt wird Prozeßgas nur zwischen den aufeinanderfolgenden Plasmaerzeugungen zweier Arbeitszyklen zugeführt. Zusätzlich wird in der Regenerationsphase des Prozeßgases zwischen zwei Plasmaerzeugungen zumindest ein Evakuieren, vorzugsweise auch das Erzeugen einer Prozeßgas-Atmosphäre eines höheren Druckes als der Plasmaerzeugungsdruck, durchgeführt.

Wie bei den beiden vorhergehenden Verfahren kann als Präcursorgas Tetrakisdimethylaminotitan (TMT) verwendet werden. Die Zusammensetzung der metallhaltigen Schicht beträgt dann bei allen vorgenannten Beispielen 40 At-% Ti, 20 At-% N, 15 At-% C und 25 At-% O. Werden andere Precursorgase verwendet, so können auch Schichten mit anderen Zusammensetzungen und Atomgehalten erzeugt werden. Diesbezüglich wird beispielsweise auf die EP 0 897 997 A1 verwiesen. Die Plasmaleistungsdichte, die bei den dargelegten Beschichtungsverfahren erforderlich ist, liegt jeweils im Bereich zwischen 10 bis 15 W/l.

Zur Beschichtung des Behältnisses wird für eine Zeitdauer von 2 Sekunden in einer Atmosphäre mit einem Druck von 1 mbar bei einer Temperatur 100° Celsius und bei einer Beladung der Atmosphäre mit 0,001 mol_{TMT}/mol H₂ ein Plasma erzeugt. Nach Beendigung der Plasmaerzeugung wird die Atmosphäre auf einen Druck von weniger als 10⁻² mbar evakuiert. Anschließend erfolgt eine Druckerhöhung der Prozeßgas-Atmosphäre auf 10 mbar. Hierzu wird Prozeßgas mit einem Volumenstrom von 10 l_{N}/H zugeführt. Anschließend wird durch Evakuieren der Druck in der Prozeßgas-Atmosphäre auf 1 mbar reduziert. Und die zum nächsten Zyklus gehörende Plasmaerzeugung kann für wiederum 2 Sekunden erfolgen. Hierbei wird bei 100 Plasmaerzeugungen (Zyklen) eine Schichtdicke von 70 nm erreicht.

Die Beschichtung auf dem Kunststoffsubstrat weist aufgrund der gleichmäßigen Beladung der Prozeßgas-Atmosphäre mit Präcursor-Substanz während der Plasmaerzeugung eine geschlossene Schicht auf der gesamten zu beschichtenden Oberfläche auf, wobei die Abweichung in der Schichtdicke höchstens 30 %, vorzugsweise höchstens 10 bis 15 % beträgt. Dies ist, wie vorstehend dargelegt, auch dann der Fall, wenn das Kunststoffsubstrat Hohlräume oder Hinterschneidungen aufweist. Je komplizierter die Geometrie des Substrates und je schlechter die Durchströmung des Substrates mit Prozeßgas ist, desto eher empfiehlt sich die Anwendung des in der Fig. 2 dargestellten Verfahrens, bei dem die Prozeßgaszufuhr lediglich zwischen den Plasmaerzeugungen erfolgt und nach dem Erzeugen eines Plasmas zunächst evakuiert wird und dann gegebenenfalls nach Erzeugen eines Prozeßgas-Überdruckes die für das Plasma erforderlichen Bedingungen eingestellt werden.

In die Metallschicht werden regelmäßig auch Kohlenstoff und Sauerstoff-Atome eingelagert. Die Einlagerung von Stickstoff erfolgt nur dann, wenn dieser in der Präcursor-Substanz enthalten ist. Die gemäß den vorstehenden Verfahren hergestellten Beschichtungen weisen auch eine sehr gute Abriebfestigkeit auf. Gleichzeitig führen sie nicht dazu, daß die Eigenschaften des das Kunstoffsubstrat bildenden Kunststoffs verändert werden. Handelt es sich bei dem Kunststoffsubstrat um ein textiles Gewebe oder dergleichen wird auch eine gute Beschichtung von Kreuzungstellen des Gewebes erreicht. Wird das beschichtete Substrat im menschlichen oder tierischen Körper verwendet, so ist vorteilhaft, daß die allenfalls sehr kleinen Fehlstellen so klein sind, daß deren Präsenz von den Zellen des Körpers nicht erkannt werden. Durch die Beschichtung kann auch eine Langzeitstabilisierung des Kunststoffsubstrats erreicht werden, und so wird beispielsweise die Hydrolyse-Empfindlichkeit von PET verringert.

Mit den vorliegenden Verfahren kann auch eine Beschichtung erzeugt werden, die eine Leitfähigkeit im Bereich von 10 1/Ω cm aufweisen, derartige Halbleiter-Oberflächen sind im menschlichen oder tierischen Körper blutverträglicher als nicht leitende Oberflächen. Gleichzeitig sind die Eigenschaften einer halbleitenden Oberfläche, wie sie beispielsweise durch Einlagerung von Stickstoff als Nitrid in die metallhaltige Schicht erreicht wird, günstiger als eine rein metallische Schicht, da sich auf letzterer eine OberflächenStromleitung behindernde Oxidschicht bilden kann.

Bevorzugte Anwendungsgebiete für die erfindungsgemäßen Verfahren bzw. Anwendungsgebiete erfindungsgemäßer dreidimensionaler Kunststoffsubstrate mit einer metallhaltigen Beschichtung sind im medizinischen Bereich:
- bei Gegenständen mit Blutkontakt:
   Gefäßprothesen; Gefäßkatheter; Filter, Membranen, Schläuche, etc. für Dialyse; Schläuche für Herz-Lungen-Maschinen, Blutbeutel
- bei Weichgewebe-Kontakt:
   Leistenhernienimplantate, nicht resorbierbares Nahtma terial und Zubehör; Silikonkissenimplantate; Hornhautersatz
- bei Knochen-Kontakt:
   Kreuzbandersatz, PTFE-Membranen
- weitere Anwendungsbeispiele:
   bei vorgefüllten Spritzen; Obturatoren und Epithesen, insbesondere für die Kieferchirurgie; urologische Katheter; Luftröhrenimplantate und Kannülen.

## Patentansprüche

1. Plasmabeschichtungsverfahren, insbesondere PACVD-Verfahren, wobei ein Substrat in eine Reaktionskammer eingebracht und eine Prozeßgas-Atmosphäre erzeugt wird, dadurch gekennzeichnet, daß für die Beschichtung eines Substrates mehrmals ein Plasma erzeugt und vor der Plasmaerzeugung jeweils eine geeignete Prozeßgas-Atmosphäre erzeugt wird.

2. Plasmabeschichtungsverfahren nach Anspruch 1, dadurch gekennzeichnet, daß die Plasma-Erzeugung jeweils für eine vorgegebene Brenndauer, insbesondere eine Brenndauer zwischen 1 Sekunde und 5 Sekunden, vorgenommen wird.

3. Plasmabeschichtungsverfahren nach Anspruch 1 oder 2, dadurch gekennzeichnet, daß zwischen 2 Plasmaerzeugungen ein Erzeugen einer geeigneten Prozeßgas-Atmosphäre durch Gasaustausch erfolgt, wobei für den Gasaustausch insbesondere eine kontinuierliche Zufuhr von Prozeßgas in die Reaktionskammer vorgenommen wird, und wobei zwischen zwei Plasma-Erzeugungen jeweils ein Zeitintervall für die Erneuerung der Prozeßgas-Atmosphäre liegt.

4. Plasmabeschichtungsverfahren nach Anspruch 3, dadurch gekennzeichnet, daß der Gasaustausch durch Abpumpen des am Ende einer Plasma-Erzeugung verbleibenden Restgases und ein anschließendes Wiederbefüllen mit Prozeßgas erfolgt, wobei vorzugsweise beim Wiederbefüllen mit Prozeßgas in der Reaktionskammer zunächst ein höherer als während der Plasmaerzeugung erwünschter Druck eingestellt und dann der Druck auf den während der Plasma-Erzeugung erwünschten Druck reduziert wird.

5. Plasmabeschichtungsverfahren nach einem der vorstehenden Ansprüche, dadurch gekennzeichnet, daß die Anzahl der Plasma-Erzeugungen abhängig von der zu erzeugenden Dicke der Schicht auf dem Substrat bestimmt wird.

6. Plasmabeschichtungsverfahren nach einem der vorstehenden Ansprüche, dadurch gekennzeichnet, daß die Temperatur in der Reaktionskammer derart niedrig ist, daß die zur Beschichtung führende Reaktion nur während der externen Erzeugung eines Plasmas stattfindet.

7. Plasmabeschichtungsverfahren nach Anspruch 6, dadurch gekennzeichnet, daß der Druck der Prozeßgas-Atmosphäre kleiner als 10 mbar, insbesondere gleich ca. 1 mbar eingestellt wird.

8. Plasmabeschichtungsverfahren nach einem der vorstehenden Ansprüche, dadurch gekennzeichnet, daß vor dem Erzeugen eines Plasmas in einer Prozeßgas-Atmosphäre ein Plasma in einer Stickstoff-Atmosphäre erzeugt wird.

9. Plasmabeschichtungsverfahren nach einem der vorstehenden Ansprüche, dadurch gekennzeichnet, daß im Substrat zumindest während der Plasma-Erzeugung Vibrationen erzeugt werden.

10. Dreidimensionales Kunststoffsubstrat (12) mit einer unmittelbar an die Kunststoffoberfläche angrenzenden metallhaltigen Beschichtung (13), wobei die Schichtdicke von 5 bis 700 nm, insbesondere zwischen 10 und 100 nm beträgt, dadurch gekennzeichnet, daß die metallhaltige Schicht eine gleichmäßige Schichtdicke aufweist und die Schichtdicken-Abweichung kleiner ± 30 % ist, wobei auf der gesamten zu beschichtenden Oberfläche eine geschlossene Schicht ausgebildet ist.

11. Dreidimensionales Kunststoffsubstrat nach Anspruch 10, dadurch gekennzeichnet, daß das Substrat (12) eine unregelmäßige Oberfläche und/oder Hinterschneidungen und/oder von Außen zugängliche Hohlräume aufweist.

12. Dreidimensionales Kunststoffsubstrat nach einem der Ansprüche 10 oder 11, dadurch gekennzeichnet, daß die Beschichtung (13) wenigstens teilweise eine Innenbeschichtung ist.

13. Dreidimensionales Kunststoffsubstrat nach einem der Ansprüche 10 bis 12, dadurch gekennzeichnet, daß das Kunststoffsubstrat (12) ein zum Kontakt mit dem menschlichen oder tierischen Körper bestimmter Gegenstand insbesondere eine zur Implantation in den menschlichen oder tierischen Körper bestimmte Prothese, insbesondere textile Prothese oder Gefäßprothese, ist.

14. Dreidimensionales Kunststoffsubstrat nach einem der Ansprüche 10 bis 13, dadurch gekennzeichnet, daß die Beschichtung diffusionsdicht, insbesondere als Diffusionsperre für Stoffe aus dem Substrat oder in das Substrat, ausgebildet ist.

15. Dreidimensionales Kunststoffsubstrat nach einem der Ansprüche 10 bis 14, dadurch gekennzeichnet, daß die elektrische Leitfähigkeit der metallhaltigen Schicht größer als 10⁻⁴ 1/Ω cm ist.

16. Dreidimensionales Kunststoffsubstrat nach einem der Ansprüche 10 bis 15, dadurch gekennzeichnet, daß die Schicht wenigstens eines der Elemente Ti, Ta, Nb, Zr, Hf und N enthält.
